# EUROPEAN PATENT APPLICATION

(11) **EP 1 164 191 A1**
(43) Date of publication of application: **19.12.2001**
(21) Application number: 00911352.3
(22) Date of filing: 24.03.2000
(51) Int. Cl.: C12N 15/12, C07K 14/705, C07K 16/28, C12P 21/02, G01N 33/50

(54) **NOVEL GUANOSINE TRIPHOSPHATE (GTP)-BINDING PROTEIN-COUPLED RECEPTOR PROTEINS, BG3**

(30) Priority: 25.03.1999 JP 8264199
(71) Applicant: BANYU PHARMACEUTICAL CO., LTD., Chuo-ku, Tokyo 103-8416 (JP)
(72) Inventor: NAKAMURA, Takao, Banyu Pharmaceutical Co., Ltd., Tsukuba-shi, Ibaraki 300-2611 (JP); OHTA, Masataka, Banyu Pharmaceutical Co., Ltd., Tsukuba-shi, Ibaraki 300-2611 (JP)
(74) Representative: VOSSIUS & PARTNER
(86) International application number: JP0001826
(87) International publication number: WO0058462

(57) **Abstract**

The present inventors isolated a cDNA which encodes a novel human G protein-coupled receptor protein, by deducing a unique region highly conserved among known G protein-coupled receptor proteins and screening cDNAs by using the probe corresponding to the region. The G protein-coupled receptor protein of the present invention can be used to screen for ligands and candidate pharmaceutical compounds capable of modulating the signal transduction mediated by the receptor.

SEQUENCE LISTING

<110> BANYU PHARMACEUTICAL CO., LTD.

<120> NOVEL GUANOSINE TRIPHOSPHATE (GTP)
      BINDING PROTEIN-COUPLED RECEPTOR PROTEIN, BG3

<130> B1-101PCT

<140>
<141>

<150> JP 1999-82641
<151> 1999-03-25

<160> 6

<170> PatentIn Ver. 2.0

<210> 1
<211> 21
<212> DNA
<213> Artificial Sequence

<220>
<223> Description of Artificial Sequence: Artificially

synthesized primer sequence

<400> 1

batngccaac ctbkccttct c                                              21

<210> 2

<211> 3117

<212> DNA

<213> Homo sapiens

<400> 2

atcagctatg tgggctgctc cctctccgtg ctctgcctgg tggccacgct ggtcaccttc 60

gccgtgctgt cctccgtgag caccatccgg aaccagcgct accacatcca cgccaacctg 120

tccttcgccg tgctggtggc ccaggtcctg ctgctcatta gtttccgcct cgagccgggc 180

acgacccccct gccaagtgat ggccgtgctc ctacactact tcttcctgag tgccttcgca 240

tggatgctgg tggaggggct gcacctctac agcatggtga tcaaggtctt tgggtcggag 300

gacagcaagc accgttacta ctatgggatg ggatggggtt ttcctcttct gatctgcatc 360

atttcactgt catttgccat ggacagttac ggaacaagca acaattgctg gctgtcgttg 420

gcgagtggcg ccatctgggc ctttgtagcc cctgccctgt ttgtcatcgt ggtcaacatt 480
ggcatcctca tcgctgtgac cagagtcatc tcacagatca gcgccgacaa ctacaagatc 540
catggagacc ccagtgcctt caagttgacg gccaaggcag tggccgtgct gctgcccatc 600
ctgggtacct cgtgggtctt tggcgtgctt gctgtcaacg gttgtgctgt ggttttccag 660
tacatgtttg ccacgctcaa ctccctgcag ggactgttca tattcctctt tcattgtctc 720
ctgaattcag aggtgagagc cgccttcaag cacaaaacca aggtctggtc gctcacgagc 780
agctccgccc gcacctccaa cgcgaagccc ttccactcgg acctcatgaa tgggacccgg 840
ccaggcatgg cctccaccaa gctcagccct tgggacaaga gcagccactc tgcccaccgc 900
gtcgacctgt cagccgtgtg agccgggagg ctgccaacca ggccaggctg cgctcagaac 960
acaccccccc aaacagaatg aaatgcccca cctttgccca tggaccctct ccttgctgct 1020
gtctggacat gggtgttgtg gccccgagac agctgtcctc ccctgtgact ctggctgtcg 1080
gagcacactg ctcagcccag cagcctgatg cccaggccag cgtgggccct cctgccttgc 1140
atccacccgt gggctgagtg acttcctcgg gggattccca ggacacagtg gcctgactgt 1200

gatggtgccc ttgagcctcc cttcatcact cagcatcaga cccagcgagg ccaggacact 1260

cggggccggt cccgcagcac caggagggga tgttcagcct ctgtgccttg gtggggcttg 1320

gggactcagg gccaaagagg tggttcaggt ccccacgcac cctcagtcag gcgcaggcag 1380

ctgggggtgt gtggggaaga gcatgcggag tccccagtgt ctgaatccac tgagtggtga 1440

gttccccaca gccggcgcta gctggtgtgt gtctctgtag gtggtgccgg cgtgggccaa 1500

cctgtgctgt gtcatcagtt gggggcccct gcccaagccg agctcgagcc gtgggcggga 1560

gtcgttgact ctccaggtga gggcgacccc tctgccctgt ccttgggggg gtcccctctg 1620

ctcacgtgaa gagccgctct gggccttgag gctgcctgat ggtgcctgtg cttgggggag 1680

cttctcggcc atccgctgtg agttttgcct ctttggaccc caattcggcc ttaagatgcc 1740

ctcctccctc gtgtgccagc ctccttggtt gttcttgggc cacaggagct ggccgtgtcc 1800

ccgcagtgcc tggtgtccag gtggaaagtg gagggcattt tccagggcac tgctttcccc 1860

agaggcttcc tcatggctca caggcactct acgaagtttc taatgggcag accacgcggc 1920

aggtagcaca gtgcgctccg tctggtcacc atgagaccga cctgcgctga gtccccactg 1980

acctggagag ggagggctgg tgacagccgt gtcttctgtg ttgagggaaa tttatggact 2040

cagattcagc cccagaggag atgggataat tgttatggac ccatgtgtgg gcatgatcct 2100

gtggaacaca ggtttgggat catagatgtg aattaagaca ccaccgagat acgggctgtg 2160

aggttcatac tgtgctgata gcactcgtgg tgtctgtgaa atgtgggtaa gacattcaaa 2220

cctggttttg atactggaaa ctcttccttt aaaactgtga ccatgatttc attcagcccc 2280

tccacacccc tatgtctgcc ttgtttcaga gtgagttttc tatggagcct gtggcccttt 2340

tgcagcccac ctggtggctt cttaatgtaa ctcttcccct ggtcgcctgg agtggaccac 2400

tcatctgcag gcctctcctg catggggagg gtaggcaggg agcagcatgt ctgcaggggt 2460

gaacctttgc tcttctgtca ggcgaggccc aggctgcacc agccacctgc cacatggtga 2520

cagtgccacg ggccctgcgt atggcccctg caaccgtgct ctggcgggca cacctggctg 2580

ctgcaggcca aggccgctgt tcagtgaaga gtcccatgtt tagtatggac taaagtccca 2640

tgtttagcca ctgccccagg ctcccgtgac cccagaaacc aggtcacatg gaccacagtg 2700

ccagatcctc atcacgccgg tgagcaccta gaagtgagaa cactgtattc ctacaatgta 2760

cacttggata tttctcctta tttagtttct agtgaaacaa atcaagtaag gaactatctt 2820

tagtttagat ggaattattt gtttttaatt gttgccgtat tcatctatat agctaatatt 2880

tcaagataag taatgaacaa aacctgtcta aacctttttgt ttccaatgaa tgaaagtcat 2940

gcactttatt tataggctct atgttttggc ttctgcagta cttttattat ctatacataa 3000

tttggccaaa aataagaaat tggaaagaat gaaatgttta gtttatagta gaagaaagat 3060

gatgacacta agttgtgaaa atatgttgtg atttttatga aataaactca tgtcctg 3117

<210> 3

<211> 21

<212> DNA

<213> Artificial Sequence

<220>

<223> Description of Artificial Sequence: Artificially

synthesized primer sequence

<400> 3

ccacgccaac ctgtccttcg c 21

<210> 4

<211> 21

<212> DNA

<213> Artificial Sequence

<220>

<223> Description of Artificial Sequence: Artificially

synthesized primer sequence

<400> 4

ctgctcgtga gcgaccagac c 21

<210> 5

<211> 5340

<212> DNA

<213> Homo sapiens

<220>

<221> CDS

<222> (517)..(3138)

<400> 5

ttgagccaga ccagaaggag ctcgagagcg gccgcaggac aagcccgagg agcaggcggg 60

cgctccaggg gaaaaccacg cacaaaacct tcttcagaga aaagggaagc tccaaacctg 120

actgagacaa acggaggctc ttgaaataaa aagaaaatac cgcaggacaa acagcctccc 180

gtccccgggc gcaggtcgcg gtcacagtgg tgacctggga ttgctttccc aggactgcga 240

gtcgggtttg ggtttctcct ccctgcattc cacagctgct ctggtcatcg caacgtgttt 300

attgatcact gaagaatctc aagttttgag acgaggaaga aacacccatt aggtctccaa 360

gacagctgtg tttcacaaac tttagggaga cagaaatttt ctcccctgga acctgtgaaa 420

atgtcccttt tccaaggaag tgaaggttaa gaggtcccgt tctcacagac cctcagtaat 480

ttcacttggc tccgagcttt gacctccgag agagcc atg gaa aag ctg ctg cgg 534
Met Glu Lys Leu Leu Arg
1               5

ctg tgc tgc tgg tac tcc tgg ctg ctg cta ttt tat tac aac ttt cag 582
Leu Cys Cys Trp Tyr Ser Trp Leu Leu Leu Phe Tyr Tyr Asn Phe Gln
            10                  15                  20

gtg cgt ggc gtc tac tcc aga tcg cag gac cat cca gga ttt cag gtg 630
Val Arg Gly Val Tyr Ser Arg Ser Gln Asp His Pro Gly Phe Gln Val
        25                  30                  35

ttg gcg tct gct tcc cat tac tgg cca ctg gag aat gtg gat ggg atc 678

Leu Ala Ser Ala Ser His Tyr Trp Pro Leu Glu Asn Val Asp Gly Ile
    40                  45                  50

cat gaa ctt cag gat aca act gga gat att gtg gaa ggg aag gtc aac 726
His Glu Leu Gln Asp Thr Thr Gly Asp Ile Val Glu Gly Lys Val Asn
55                  60                  65                  70

aaa ggc att tac ctg aaa gag gaa aag gga gtc acg ctt ctc tat tac 774
Lys Gly Ile Tyr Leu Lys Glu Glu Lys Gly Val Thr Leu Leu Tyr Tyr
                75                  80                  85

ggc agg tac aac agc tcc tgc atc agc aag cca gag cag tgt ggc cct 822
Gly Arg Tyr Asn Ser Ser Cys Ile Ser Lys Pro Glu Gln Cys Gly Pro
            90                  95                  100

gaa ggg gtc acg ttt tct ttt ttc tgg aag aca caa gga gaa cag tct 870
Glu Gly Val Thr Phe Ser Phe Phe Trp Lys Thr Gln Gly Glu Gln Ser
        105                 110                 115

aga cca atc cct tct gcg tat ggg gga cag gtc atc tcc aat ggg ttc 918
Arg Pro Ile Pro Ser Ala Tyr Gly Gly Gln Val Ile Ser Asn Gly Phe
    120                 125                 130

aaa gtc tgc tcc agc ggt ggc aga ggc tct gtg gag ctg tac acg cgg 966
Lys Val Cys Ser Ser Gly Gly Arg Gly Ser Val Glu Leu Tyr Thr Arg
135                 140                 145                 150

gac aat tcc atg aca tgg gag gcc tcc ttc agc ccc cca ggc ccc tat 1014
Asp Asn Ser Met Thr Trp Glu Ala Ser Phe Ser Pro Pro Gly Pro Tyr
                155                 160                 165

tgg act cat gtc cta ttt aca tgg aaa tcc aag gag ggc ctg aaa gtc 1062
Trp Thr His Val Leu Phe Thr Trp Lys Ser Lys Glu Gly Leu Lys Val
            170                 175                 180

tac gtc aac ggg acc ctg agc acc tct gat ccg agt gga aaa gtg tct 1110
Tyr Val Asn Gly Thr Leu Ser Thr Ser Asp Pro Ser Gly Lys Val Ser
        185                 190                 195

cgt gac tat gga gag tcc aac gtc aac ctc gtg ata ggg tct gag cag 1158
Arg Asp Tyr Gly Glu Ser Asn Val Asn Leu Val Ile Gly Ser Glu Gln
    200                 205                 210

gac cag gcc aag tgt tat gag aac ggt gct ttc gat gag ttc atc atc 1206
Asp Gln Ala Lys Cys Tyr Glu Asn Gly Ala Phe Asp Glu Phe Ile Ile
215                 220                 225                 230

tgg gag cgg gct ctg act ccg gat gag atc gcc atg tac ttc act gct 1254
Trp Glu Arg Ala Leu Thr Pro Asp Glu Ile Ala Met Tyr Phe Thr Ala
                235                 240                 245

gcc att gga aag cat gct tta ttg tct tca acg ctg cca agc ctc ttc 1302

Ala Ile Gly Lys His Ala Leu Leu Ser Ser Thr Leu Pro Ser Leu Phe
            250                 255                 260

atg aca tcc aca gca agc ccc gtg atg ccc aca gat gcc tac cat ccc 1350
Met Thr Ser Thr Ala Ser Pro Val Met Pro Thr Asp Ala Tyr His Pro
        265                 270                 275

atc ata acc aac ctg aca gaa gag aga aaa acc ttc caa agt ccc gga 1398
Ile Ile Thr Asn Leu Thr Glu Glu Arg Lys Thr Phe Gln Ser Pro Gly
    280                 285                 290

gtg ata ctg agt tac ctc caa aat gta tcc ctc agc tta ccc agt aag 1446
Val Ile Leu Ser Tyr Leu Gln Asn Val Ser Leu Ser Leu Pro Ser Lys
295                 300                 305                 310

tcc ctc tcg gag cag aca gcc ttg aat ctc acc aag acc ttc tta aaa 1494
Ser Leu Ser Glu Gln Thr Ala Leu Asn Leu Thr Lys Thr Phe Leu Lys
                315                 320                 325

gcc gtg gga gag atc ctt cta ctg cct ggt tgg att gct ctg tca gag 1542
Ala Val Gly Glu Ile Leu Leu Leu Pro Gly Trp Ile Ala Leu Ser Glu
            330                 335                 340

gac agc gcc gtg gta ctg agt ctc atc gac act att gac acc gtc atg 1590
Asp Ser Ala Val Val Leu Ser Leu Ile Asp Thr Ile Asp Thr Val Met
        345                 350                 355

ggc cat gta tcc tcc aac ctg cac ggc agc acg ccc cag gtc acc gtg 1638
Gly His Val Ser Ser Asn Leu His Gly Ser Thr Pro Gln Val Thr Val
    360                 365                 370

gag ggc tcc tct gcc atg gca gag ttt tcc gtg gcc aaa atc ctg ccc 1686
Glu Gly Ser Ser Ala Met Ala Glu Phe Ser Val Ala Lys Ile Leu Pro
375                 380                 385                 390

aag acc gtg aat tcc tcc cat tac cgc ttc ccg gcc cac ggg cag agc 1734
Lys Thr Val Asn Ser Ser His Tyr Arg Phe Pro Ala His Gly Gln Ser
                395                 400                 405

ttc atc cag atc ccc cac gag gcc ttc cac agg cac gcc tgg agc acc 1782
Phe Ile Gln Ile Pro His Glu Ala Phe His Arg His Ala Trp Ser Thr
            410                 415                 420

gtc gtg ggt ctg ctg tac cac agc atg cac tac tac ctg aac aac atc 1830
Val Val Gly Leu Leu Tyr His Ser Met His Tyr Tyr Leu Asn Asn Ile
        425                 430                 435

tgg ccc gcc cac acc aag atc gcg gag gcc atg cat cac cag gac tgc 1878
Trp Pro Ala His Thr Lys Ile Ala Glu Ala Met His His Gln Asp Cys
    440                 445                 450

ctg ctg ttc gcc acc agc cac ctg att tcc ctg gag gtg tcc cca cca 1926

Leu Leu Phe Ala Thr Ser His Leu Ile Ser Leu Glu Val Ser Pro Pro
455                 460                 465                 470

ccc acc ctg tct cag aac ctg tcg ggc tct cca ctc att acg gtc cac     1974
Pro Thr Leu Ser Gln Asn Leu Ser Gly Ser Pro Leu Ile Thr Val His
                475                 480                 485

ctc aag cac aga ttg aca cgt aag cag cac agt gag gcc acc aac agc     2022
Leu Lys His Arg Leu Thr Arg Lys Gln His Ser Glu Ala Thr Asn Ser
            490                 495                 500

agc aac cga gtc ttc gtg tac tgc gcc ttc ctg gac ttc agc tcc gga     2070
Ser Asn Arg Val Phe Val Tyr Cys Ala Phe Leu Asp Phe Ser Ser Gly
        505                 510                 515

gaa ggg gtc tgg tcg aac cac ggc tgt gcg ctc acg aga gga aac ctc     2118
Glu Gly Val Trp Ser Asn His Gly Cys Ala Leu Thr Arg Gly Asn Leu
    520                 525                 530

acc tac tcc gtc tgc cgc tgc act cac ctc acc aac ttt gcc atc ctc     2166
Thr Tyr Ser Val Cys Arg Cys Thr His Leu Thr Asn Phe Ala Ile Leu
535                 540                 545                 550

atg cag gtg gtc ccg ctg gag ctt gca cgc gga cac cag gtg gcg ctg     2214
Met Gln Val Val Pro Leu Glu Leu Ala Arg Gly His Gln Val Ala Leu
                555                 560                 565

tcg tct atc agc tat gtg ggc tgc tcc ctc tcc gtg ctc tgc ctg gtg 2262
Ser Ser Ile Ser Tyr Val Gly Cys Ser Leu Ser Val Leu Cys Leu Val
            570                 575                 580

gcc acg ctg gtc acc ttc gcc gtg ctg tcc tcc gtg agc acc atc cgg 2310
Ala Thr Leu Val Thr Phe Ala Val Leu Ser Ser Val Ser Thr Ile Arg
        585                 590                 595

aac cag cgc tac cac atc cac gcc aac ctg tcc ttc gcc gtg ctg gtg 2358
Asn Gln Arg Tyr His Ile His Ala Asn Leu Ser Phe Ala Val Leu Val
    600                 605                 610

gcc cag gtc ctg ctg ctc att agt ttc cgc ctc gag ccg ggc acg acc 2406
Ala Gln Val Leu Leu Leu Ile Ser Phe Arg Leu Glu Pro Gly Thr Thr
615                 620                 625                 630

ccc tgc caa gtg atg gcc gtg ctc cta cac tac ttc ttc ctg agt gcc 2454
Pro Cys Gln Val Met Ala Val Leu Leu His Tyr Phe Phe Leu Ser Ala
                635                 640                 645

ttc gca tgg atg ctg gtg gag ggg ctg cac ctc tac agc atg gtg atc 2502
Phe Ala Trp Met Leu Val Glu Gly Leu His Leu Tyr Ser Met Val Ile
            650                 655                 660

aag gtc ttt ggg tcg gag gac agc aag cac cgt tac tac tat ggg atg 2550

Lys Val Phe Gly Ser Glu Asp Ser Lys His Arg Tyr Tyr Tyr Gly Met
        665                 670                 675

gga tgg ggt ttt cct ctt ctg atc tgc atc att tca ctg tca ttt gcc 2598
Gly Trp Gly Phe Pro Leu Leu Ile Cys Ile Ile Ser Leu Ser Phe Ala
    680                 685                 690

atg gac agt tac gga aca agc aac aat tgc tgg ctg tcg ttg gcg agt 2646
Met Asp Ser Tyr Gly Thr Ser Asn Asn Cys Trp Leu Ser Leu Ala Ser
695                 700                 705                 710

ggc gcc atc tgg gcc ttt gta gcc cct gcc ctg ttt gtc atc gtg gtc 2694
Gly Ala Ile Trp Ala Phe Val Ala Pro Ala Leu Phe Val Ile Val Val
                715                 720                 725

aac att ggc atc ctc atc gct gtg acc aga gtc atc tca cag atc agc 2742
Asn Ile Gly Ile Leu Ile Ala Val Thr Arg Val Ile Ser Gln Ile Ser
            730                 735                 740

gcc gac aac tac aag atc cat gga gac ccc agt gcc ttc aag ttg acg 2790
Ala Asp Asn Tyr Lys Ile His Gly Asp Pro Ser Ala Phe Lys Leu Thr
        745                 750                 755

gcc aag gca gtg gcc gtg ctg ctg ccc atc ctg ggt acc tcg tgg gtc 2838
Ala Lys Ala Val Ala Val Leu Leu Pro Ile Leu Gly Thr Ser Trp Val
    760                 765                 770

ttt ggc gtg ctt gct gtc aac ggt tgt gct gtg gtt ttc cag tac atg 2886
Phe Gly Val Leu Ala Val Asn Gly Cys Ala Val Val Phe Gln Tyr Met
775                 780                 785                 790

ttt gcc acg ctc aac tcc ctg cag gga ctg ttc ata ttc ctc ttt cat 2934
Phe Ala Thr Leu Asn Ser Leu Gln Gly Leu Phe Ile Phe Leu Phe His
                795                 800                 805

tgt ctc ctg aat tca gag gtg aga gcc gcc ttc aag cac aaa acc aag 2982
Cys Leu Leu Asn Ser Glu Val Arg Ala Ala Phe Lys His Lys Thr Lys
            810                 815                 820

gtc tgg tcg ctc acg agc agc tcc gcc cgc acc tcc aac gcg aag ccc 3030
Val Trp Ser Leu Thr Ser Ser Ser Ala Arg Thr Ser Asn Ala Lys Pro
        825                 830                 835

ttc cac tcg gac ctc atg aat ggg acc cgg cca ggc atg gcc tcc acc 3078
Phe His Ser Asp Leu Met Asn Gly Thr Arg Pro Gly Met Ala Ser Thr
    840                 845                 850

aag ctc agc cct tgg gac aag agc agc cac tct gcc cac cgc gtc gac 3126
Lys Leu Ser Pro Trp Asp Lys Ser Ser His Ser Ala His Arg Val Asp
855                 860                 865                 870

ctg tca gcc gtg tgagccggga ggctgccaac caggccaggc tgcgctcaga 3178

Leu Ser Ala Val

acacaccccc cccaaacaga atgaaatgcc ccacctttgc ccatggaccc tctccttgct 3238

gctgtctgga catgggtgtt gtggccccga gacagctgtc ctcccctgtg actctggctg 3298

tcggagcaca ctgctcagcc cagcagcctg atgcccaggc cagcgtgggc cctcctgcct 3358

tgcatccacc cgtgggctga gtgacttcct cgggggattc ccaggacaca gtggcctgac 3418

tgtgatggtg cccttgagcc tcccttcatc actcagcatc agacccagcg aggccaggac 3478

actcggggcc ggtcccgcag caccaggagg ggatgttcag cctctgtgcc ttggtggggc 3538

ttggggactc agggccaaag aggtggttca ggtccccacg caccctcagt caggcgcagg 3598

cagctggggg tgtgtgggga agagcatgcg gagtccccag tgtctgaatc cactgagtgg 3658

tgagttcccc acagccggcg ctagccgtgg tgtgtgtctc tgtaggtggt gccggcgtgg 3718

gccaacctgt gctgtgtcat cagttggggg cccctgccca agccgagctc gagccgtggg 3778

cgggagtcgt tgactctcca ggtgagggcg acccctctgc cctgtccttg gggggtcccc 3838

ctctgctcac gtgaagagcc gctctgggcc ttgaggctgc ctgatggtgc ctgtgcttgg 3898

gggagcttct cggccatccg ctgtgagttt tgcctctttg gaccccaatt cggccttaag 3958

atgccctcct ccctcgtgtg ccagcctcct tggttgttct tgggccacag gagctcgccg 4018

tgtccccgca gtgcctggtg tccaggtgga aagtggaggg cattttccag ggcactgctt 4078

tccccagagg cttcctcatg gctcacaggc actctacgaa gtttctaatg ggcagaccac 4138

gcggcaggta gcacagtgcg ctccgtctgg tcaccatgag accgacctgc gctgagtccc 4198

cactgacctg gagagggagg gctggtgaca gccgtgtctt ctgtgttgag ggaaatttat 4258

ggactcagac tcagccccag aggagatggg ataattgtta tggacccatg tgtgggcatg 4318

atcctgtgga acacaggttt gggatcatag atgtgaatta agacaccacc gagatacggg 4378

ctgtgaggtt cataccgtgc tgatagcact cgtggtgtct gtgaaatgtg ggtaagacat 4438

tcaaacctgg ttttgatact ggaaactctt cctttaaaac tgtgaccatg atttcattca 4498

gcccctccac acccctatgt ctgccttgtt tcagagtgag ttttctatgg agcctgtggc 4558

ccttttgcag cccacctggt ggcttcttaa tgtaactctt cccctggtcg cctggagtgg 4618

accactcatc tgcaggcctc tcctgcatgg ggagggtagg cagggagcag catgtctgca 4678

ggggtgaacc tttgctcttc tgtcaggcga ggcccaagct gcaccagcca cctgccacat 4738

ggtgacagtg ccacgggccc tgcgtatggc ccctgcaacc gtgctctggc gggcacacct 4798

ggctgctgca ggccaaggcc gctgttcagt gaagagtccc atgtttagta tggactaaag 4858

tcccatgttt agccactgcc ccaggctccc gtgaccccag aaaccaggtc acatggacca 4918

cagtgccaga tcctcatcac gccggtgagc acctagaagt gagaacactg tattcctaca 4978

atgtacactt ggatatttct ccttatttag tttctagtga aacaaatcaa gtaaggaact 5038

atctttagtt tagatggaat tatttgtttt taattgttgc cgtattcatc tatatagcta 5098

atatttcaag ataagtaatg aacaaaacct gtctaaacct tttgtttcca atgaatgaaa 5158

gtcatgcact ttatttatag gctctatgtt ttggcttctg cagtactttt attatctata 5218

cataatttgg ccaaaaataa gaaattggaa agaatgaaat gtttagttta tagtagaaga 5278

aagatgatga cactaagttg tgaaaatatg ttgtgatttt tatgaaataa actcatgtcc 5338

tg 5340

<210> 6

<211> 874

<212> PRT

<213> Homo sapiens

<400> 6

Met Glu Lys Leu Leu Arg Leu Cys Cys Trp Tyr Ser Trp Leu Leu Leu
1               5                   10                  15

Phe Tyr Tyr Asn Phe Gln Val Arg Gly Val Tyr Ser Arg Ser Gln Asp
            20                  25                  30

His Pro Gly Phe Gln Val Leu Ala Ser Ala Ser His Tyr Trp Pro Leu
        35                  40                  45

Glu Asn Val Asp Gly Ile His Glu Leu Gln Asp Thr Thr Gly Asp Ile
    50                  55                  60

Val Glu Gly Lys Val Asn Lys Gly Ile Tyr Leu Lys Glu Glu Lys Gly
65                  70                  75                  80

Val Thr Leu Leu Tyr Tyr Gly Arg Tyr Asn Ser Ser Cys Ile Ser Lys
                85                  90                  95

Pro Glu Gln Cys Gly Pro Glu Gly Val Thr Phe Ser Phe Phe Trp Lys
            100                 105                 110

Thr Gln Gly Glu Gln Ser Arg Pro Ile Pro Ser Ala Tyr Gly Gly Gln
        115                 120                 125

Val Ile Ser Asn Gly Phe Lys Val Cys Ser Ser Gly Gly Arg Gly Ser
    130                 135                 140

Val Glu Leu Tyr Thr Arg Asp Asn Ser Met Thr Trp Glu Ala Ser Phe
145                 150                 155                 160

Ser Pro Pro Gly Pro Tyr Trp Thr His Val Leu Phe Thr Trp Lys Ser
                165                 170                 175

Lys Glu Gly Leu Lys Val Tyr Val Asn Gly Thr Leu Ser Thr Ser Asp
            180                 185                 190

Pro Ser Gly Lys Val Ser Arg Asp Tyr Gly Glu Ser Asn Val Asn Leu
        195                 200                 205

Val Ile Gly Ser Glu Gln Asp Gln Ala Lys Cys Tyr Glu Asn Gly Ala
    210                 215                 220

Phe Asp Glu Phe Ile Ile Trp Glu Arg Ala Leu Thr Pro Asp Glu Ile
225                 230                 235                 240

Ala Met Tyr Phe Thr Ala Ala Ile Gly Lys His Ala Leu Leu Ser Ser
                245                 250                 255

Thr Leu Pro Ser Leu Phe Met Thr Ser Thr Ala Ser Pro Val Met Pro
            260                 265                 270
Thr Asp Ala Tyr His Pro Ile Ile Thr Asn Leu Thr Glu Glu Arg Lys
        275                 280                 285
Thr Phe Gln Ser Pro Gly Val Ile Leu Ser Tyr Leu Gln Asn Val Ser
    290                 295                 300
Leu Ser Leu Pro Ser Lys Ser Leu Ser Glu Gln Thr Ala Leu Asn Leu
305                 310                 315                 320
Thr Lys Thr Phe Leu Lys Ala Val Gly Glu Ile Leu Leu Leu Pro Gly
                325                 330                 335
Trp Ile Ala Leu Ser Glu Asp Ser Ala Val Val Leu Ser Leu Ile Asp
            340                 345                 350
Thr Ile Asp Thr Val Met Gly His Val Ser Ser Asn Leu His Gly Ser
        355                 360                 365
Thr Pro Gln Val Thr Val Glu Gly Ser Ser Ala Met Ala Glu Phe Ser
    370                 375                 380
Val Ala Lys Ile Leu Pro Lys Thr Val Asn Ser Ser His Tyr Arg Phe

385                 390                 395                 400

Pro Ala His Gly Gln Ser Phe Ile Gln Ile Pro His Glu Ala Phe His
                405                 410                 415

Arg His Ala Trp Ser Thr Val Val Gly Leu Leu Tyr His Ser Met His
            420                 425                 430

Tyr Tyr Leu Asn Asn Ile Trp Pro Ala His Thr Lys Ile Ala Glu Ala
        435                 440                 445

Met His His Gln Asp Cys Leu Leu Phe Ala Thr Ser His Leu Ile Ser
    450                 455                 460

Leu Glu Val Ser Pro Pro Pro Thr Leu Ser Gln Asn Leu Ser Gly Ser
465                 470                 475                 480

Pro Leu Ile Thr Val His Leu Lys His Arg Leu Thr Arg Lys Gln His
                485                 490                 495

Ser Glu Ala Thr Asn Ser Ser Asn Arg Val Phe Val Tyr Cys Ala Phe
            500                 505                 510

Leu Asp Phe Ser Ser Gly Glu Gly Val Trp Ser Asn His Gly Cys Ala
        515                 520                 525

Leu Thr Arg Gly Asn Leu Thr Tyr Ser Val Cys Arg Cys Thr His Leu
    530                 535                 540
Thr Asn Phe Ala Ile Leu Met Gln Val Val Pro Leu Glu Leu Ala Arg
545                 550                 555                 560
Gly His Gln Val Ala Leu Ser Ser Ile Ser Tyr Val Gly Cys Ser Leu
                565                 570                 575
Ser Val Leu Cys Leu Val Ala Thr Leu Val Thr Phe Ala Val Leu Ser
            580                 585                 590
Ser Val Ser Thr Ile Arg Asn Gln Arg Tyr His Ile His Ala Asn Leu
        595                 600                 605
Ser Phe Ala Val Leu Val Ala Gln Val Leu Leu Leu Ile Ser Phe Arg
    610                 615                 620
Leu Glu Pro Gly Thr Thr Pro Cys Gln Val Met Ala Val Leu Leu His
625                 630                 635                 640
Tyr Phe Phe Leu Ser Ala Phe Ala Trp Met Leu Val Glu Gly Leu His
                645                 650                 655
Leu Tyr Ser Met Val Ile Lys Val Phe Gly Ser Glu Asp Ser Lys His
            660                 665                 670

Arg Tyr Tyr Tyr Gly Met Gly Trp Gly Phe Pro Leu Leu Ile Cys Ile
        675                 680                 685
Ile Ser Leu Ser Phe Ala Met Asp Ser Tyr Gly Thr Ser Asn Asn Cys
    690                 695                 700
Trp Leu Ser Leu Ala Ser Gly Ala Ile Trp Ala Phe Val Ala Pro Ala
705                 710                 715                 720
Leu Phe Val Ile Val Val Asn Ile Gly Ile Leu Ile Ala Val Thr Arg
                725                 730                 735
Val Ile Ser Gln Ile Ser Ala Asp Asn Tyr Lys Ile His Gly Asp Pro
            740                 745                 750
Ser Ala Phe Lys Leu Thr Ala Lys Ala Val Ala Val Leu Leu Pro Ile
        755                 760                 765
Leu Gly Thr Ser Trp Val Phe Gly Val Leu Ala Val Asn Gly Cys Ala
    770                 775                 780
Val Val Phe Gln Tyr Met Phe Ala Thr Leu Asn Ser Leu Gln Gly Leu
785                 790                 795                 800
Phe Ile Phe Leu Phe His Cys Leu Leu Asn Ser Glu Val Arg Ala Ala

                805                 810                 815

Phe Lys His Lys Thr Lys Val Trp Ser Leu Thr Ser Ser Ser Ala Arg

            820                 825                 830

Thr Ser Asn Ala Lys Pro Phe His Ser Asp Leu Met Asn Gly Thr Arg

        835                 840                 845

Pro Gly Met Ala Ser Thr Lys Leu Ser Pro Trp Asp Lys Ser Ser His

    850                 855                 860

Ser Ala His Arg Val Asp Leu Ser Ala Val

865                 870

## Description

### Technical Field

The present invention relates to a novel G protein-coupled receptor protein that is expressed in different tissues, DNA encoding the protein, methods for producing the protein, and methods of using the protein and DNA.

### Background Art

G protein-coupled receptors (guanosine triphosphate binding protein-coupled receptors) are ubiquitously expressed in mammals, and are thought to play an important biological role.

Generally, G protein-coupled receptor proteins share amino acid sequence similarities to some degree and form several receptor superfamilies. For example, a group of receptor families represented by Class B have peptide ligands and are involved in intracorporeal regulation dependent on various hormones.

α-Latrotoxin receptor is known as one of such G protein-coupled receptor proteins (Valery, G.K. et al., Neuron 18: 925-937 (1997)). α-Latrotoxin is a neurotoxin present in the venome gland of black widow spiders, and it stimulates neurotransmitter release by acting on the presynaptic membrane of the nerve ending. α-Latrotoxin causes depletion of acetylcholine at the neuromuscular junction and facilitates the release of adrenaline from sympathetic nerve terminals, thereby resulting in a number of symptoms including topical pyrexia, pain, swelling of the regional lymph node, abdominal muscle rigidity, emesis, hypertension, tachycardia, excessive perspiration on the face, dysapnea and logopathy. The mortality rate due to this toxin reaches 3 to 12%.

As inferred from the neurotoxic action of α-Latrotoxin, α-Latrotoxin receptors may have peptide ligands and may play a role in secretion of certain neurotransmitters. Furthermore, judging from the suggested involvement of α-Latrotoxin receptors in promoting insulin secretion and such (Jochen, L. et al., EMBO J. 17:648-657 (1998)), α-Latrotoxin receptors are not only involved in neurotransmitter secretion, but are also thought to be receptor proteins involved in secretion of hormones that are widely related to intracorporeal regulation.

One of the pathways that regulates biological functions by means of hormones, neurotransmitters and G protein-coupled receptors is the hypothalamo-hypophysial system. In this system, hypothalamic hormones regulate the secretion of pituitary hormones from the pituitary gland, and the pituitary hormones released in the blood mediate the functional regulation of target cells and organs. For instance, functional regulations that are essential for living organisms, such as maintenance of homeostasis and regulation of the development and growth of the reproductive system and individuals, are conducted by way of this pathway.

Representative hypothalamic hormones are TRH, CRF, GRF, somatostatin, and such, and representative pituitary hormones are TSH, ACTH, FSH, LH, prolactin, growth hormone, oxytocin, vasopressin, etc. In particular, the secretion of pituitary hormones is regulated by the positive or negative feedback mechanisms, mediated by peripheral hormones secreted from endocrine glands and hypothalamic hormones.

It is known that these hormones and corresponding receptors are not only localized in the hypothalamo-hypophysial system but are also distributed widely in the brain. Further, they are distributed in a similar manner in the peripheral tissues and are thought to have an important function.

For example, the pancreas plays an important role in glucose metabolism by secreting glucagon and insulin as well as digestive juice. Insulin is secreted from β cells of the pancreas, and the secretion is enhanced primarily by glucose. However, various receptors exist on the β cells, and insulin secretion is known to be regulated by a variety of factors other than glucose, including peptide hormones (galanin, somatostatin, gastrin, selectin, gastric inhibitory polypeptide, glucagon, etc.) , sugars (mannose, etc.) , amino acids, neurotransmitters, and so on.

In the digestive organs, including the stomach, small intestine, and such, food is digested and absorbed by the action of various digestive juices secreted under the control of a number of hormones, hormone-like substances, neurotransmitters, biologically active substances, and so on, including gastrin, selectin, glucagon, gastrin-releasing peptide, vasoactive small-intestinal peptide, acetylcholine, noradrenaline, serotonin, etc. Secretion of these substances is considered to be regulated by their corresponding receptors present in the stomach, small intestine, etc.

Additionally, in the cardiovascular and respiratory systems, represented by the heart and lung, contraction and relaxation of cardiac and vascular smooth muscles, control of blood pressure, and such are strictly regulated under the control of neurotransmitters, hormones, biological active substances, etc.

As described above, a variety of receptor proteins for hormones and neurotransmitters are present in the peripheral tissues, as well as in the brain and pituitary gland, and are thought to play important roles in functional regulations in those tissues.

### Disclosure of the Invention

The object of the present invention is to provide a novel G protein-coupled receptor protein, DNA encoding the protein, as well as methods for producing the G protein-coupled receptor protein, and methods for using the protein and DNA.

To isolate the DNA encoding a novel G protein-coupled receptor protein, the inventors screened a human fetal brain cDNA library, based on the Biotin-avidin capture method and using a probe specific for the transmembrane domain of G protein-coupled receptor proteins. As a result, the inventors obtained a novel cDNA fragment encoding a protein with an amino acid sequence that exhibits partial homology with those sequences of known G protein-coupled receptor proteins. The cDNA fragment thus obtained was further used to prepare a probe, which was then used to screen a human heart cDNA library. Consequently, the inventors succeeded in isolating the cDNA that contained a full-length translation frame (open reading frame) (hereinafter referred to as "BG3").

Analysis of the expression of this gene, by Northern hybridization using RNAs derived from human tissues, revealed that "BG3" is expressed in various tissues; in particular, strong expression was observed in the heart, placenta and lung. Southern hybridization, using human "BG3" as the probe, led to the detection of signals in genomic DNAs from comprehensive species of mammals, suggesting that the "BG3" gene is highly conserved among mammals and has an important function.

The novel G protein-coupled receptor protein, "BG3", is a relatively large protein compared to other known G protein-coupled receptor proteins, and shows an identity of 24 to 30% and a similarity of 38 to 50% in the transmembrane domain of the protein to the receptor family group classified into Class B receptors, represented by calcitonin receptors, parathyroid hormone (PTH) receptors, glucagon receptors, etc. On the other hand, it shows no homology with any known proteins at its N-terminal extracellular domain. The α-Latrotoxin receptor mentioned above is one of the proteins that shows relatively high amino acid sequence homology with the "BG3" protein of the present invention. The "BG3" receptor protein foundby the inventors is thought to be a receptor related to hormone secretion, widely involved in intracorporeal regulation including neurotransmitter secretion.

The "BG3" protein may be advantageously used to screen for its ligands, as well as to screen for candidate pharmaceutical compounds capable of regulating signal transduction from this protein, by utilizing the binding activity or the cell stimulatory activity as an index.

Consequently, the present invention relates to a novel G protein-coupled receptor protein, and the DNA encoding the protein, as well as to methods of screening for candidate ligands and pharmaceutical compounds using the same. More specifically, the present invention provides the following:
(1) DNA encoding a guanosine triphosphate binding protein-coupled receptor protein, wherein said DNA is selected from one of the following:
   (a) a DNA encoding a protein consisting of the amino acid sequence of SEQ ID NO: 6;
   (b) a DNA comprising the coding region of the nucleotide sequence of SEQ ID NO: 5;
   (c) a DNA encoding a protein consisting of the amino acid sequence of SEQ ID NO: 6 in which one or more amino acids are substituted, deleted, inserted and/or added; and
   (d) a DNA hybridizing under stringent conditions with a DNA consisting of the nucleotide sequence of SEQ ID NO: 5;
(2) a DNA encoding a partial peptide of the guanosine triphosphate binding protein-coupled receptor protein consisting of the amino acid sequence of SEQ ID NO: 6;
(3) a vector containing the DNA of (1) or (2) ;
(4) a transformant having the DNA of (1) or (2), or the vector of (3) ;
(5) a protein or peptide encoded by the DNA of (1) or (2) ;
(6) a method for producing the protein or peptide of (5), which comprises the steps of culturing the transformant of (4) and collecting the expressed protein from the transformant or culture supernatant thereof;
(7) a method of screening for a ligand that binds to the protein or peptide of (5), wherein the method comprises the steps of:
   (a) contacting the test compound with a protein or peptide of (5) ;
      and
   (b) selecting the compound that binds to said protein or peptide;
(8) a method of screening for a compound that has an inhibitory activity on the binding between the protein or peptide of (5) and the ligand thereof, wherein the method comprises the steps of:
   (a) detecting the binding activity of a protein or peptide of (5) to its ligand by contacting said protein or peptide with the ligand in the presence of the test compound; and
   (b) selecting a compound that reduces the binding activity of said protein or peptide to the ligand, by comparing the binding activity detected in step (a) with that detected in the absence of the test compound;
(9) a kit for screening for a compound that inhibits the binding of a protein or peptide of (5) to its ligand, wherein,said kit comprises the protein or peptide of (5) ;
(10) an antibody that binds to the protein of (5); and
(11) a DNA hybridizing to the DNA consisting of the nucleotide sequence of SEQ ID NO: 5 or to the complementary strand thereof, wherein said DNA has a length of at least 15 nucleotides.

As used herein, the term "G protein-coupled receptor protein" refers to a receptor protein that mediates intracellular signaling through activation of the G protein. The term "ligand" used herein refers to a naturally occurring compound capable of inducing signal transduction by binding to the G protein-coupled receptor protein, . The term "agonist", as used herein, refers to both compounds that naturally occur and those that are artificially synthesized, wherein an agonist has the same biological activity as the ligand of a G protein-coupled receptor protein. As used herein, the term "antagonist" refers to both compoundsthat naturally occur and those that are artificially synthesized, wherein an antagonist is capable of inhibiting the biological activity of a ligand of a G protein-coupled receptor protein. In addition, the "protein" and "peptide" of the present invention includes the salt forms thereof.

The present invention provides a novel G protein-coupled receptor protein. The nucleotide sequence of the cDNA of the human G protein-coupled receptor protein "BG3", which was isolated by the present inventors, is shown in SEQ ID NO: 5, and the amino acid sequence of the "BG3" protein encoded by the cDNA is shown in SEQ ID NO: 6.

The gene encoding the "BG3" protein was isolated from a human fetal brain cDNA library by the biotin-avidin capture method using the Fg probe.

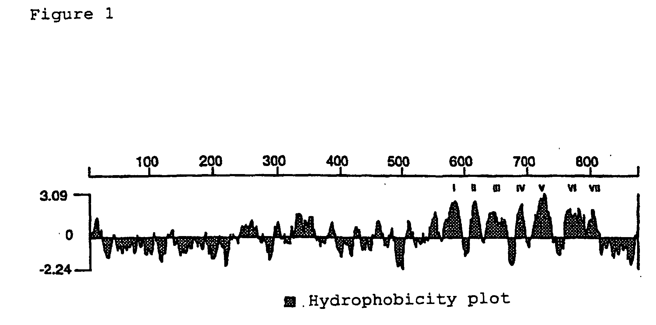

The human "BG3" protein of the present invention contains an open reading frame encoding a protein consisting of 874 amino acid residues. The result of hydrophobicity analysis revealed that the "BG3" protein possesses seven hydrophobic domains characteristic of G protein-coupled receptor proteins (Fig. 1).

The "BG3" protein shares homology with the rat Ca⁺⁺-independent α-latrotoxin receptor, i.e. showing 38% identity and 56% similarity extending over 328 amino acid residues. The "BG3" protein also shows 27% identity and 49% similarity with several corticotropin releasing factor receptors (CRF-R) over the stretch of 269 amino acid residues.

Expression of the receptor mRNA encoding the "BG3" protein was investigated in the human tissues, and the signals were detected in all the tissues examined (heart, brain, placenta, lung, liver, skeletal muscle, kidney and pancreas). Among these tissues, especially intense signals were detected in heart, placenta arid lung.

These facts indicate that the "BG3" protein belongs to the G protein-coupled receptor protein family. Furthermore, the fact that the "BG3" protein is a G protein-coupled receptor protein indicates that this protein mediates signal transduction via the activation of G proteins, particularly by the action of its ligand.

The endogenous ligand for the "BG3" receptor protein has not yet been identified. The protein of the present invention may be used to screen for a ligand useful as a pharmaceutical product or a compound that inhibits the binding of the ligand.

Potential ligands for the protein of the present invention include, but are not limited to, PTH (parathyroid hormone), calcitonin, CGRP (calcitonin gene related peptide) , giucagon, selectin, adrenomedullin, serotonin, adrenaline and noradrenaline, galanin, somatostatin, and chemokines.

Aberrations in the signal transduction mediated by the protein of the present invention are thought to result in various diseases. Therefore, compounds that activate the G protein-coupled receptor protein of the present invention or that can inhibit the function of the G protein-coupled receptor protein are expected to be useful as pharmaceuticals. When a compound that activates the G protein-coupled receptor protein of the present invention is used for prophylactic or therapeutic purposes, diseases to be treated may include asthma, Parkinson's disease, acute heart failure, chronic heart failure, congenital heart disease, diabetes, renal failure, cancer, metastatic cancer, hypotension (or hypertension), urine retention, osteoporosis, and so on.

On the other hand, diseases that may be treated with compounds that inhibit activation of the G protein-coupled receptor protein of the present invention for prophylactic and therapeutic purposes may include hypertension (or hypotension), angina pectoris, myocardial infarction, cerebral infarction, diabetes, renal failure, cancer, ulcer, asthma, allergy, benign prostatomegaly, psychopathic and neurological disorders including schizophrenia, manic excitement, depression, delirium, dementia and serious mental retardation, dyskinesia including Huntington's disease and Gilles de la Tourette's syndrome, and so on. Additionally, it is conceivable that a compound that inhibits the G protein-coupled receptor may be beneficial for the reversal of endogenous anorexia and adjustment of pathological starvation.

The protein of the present invention may be prepared as a recombinant protein, by means of recombinant DNA technology, or as a naturally occurring protein. A naturally occurring protein may be prepared, for instance, by performing affinity chromatography on the extract of tissues suspected to express the "BG3" protein (e.g. heart, placenta and lung) with the antibody raised against the "BG3" protein described below. On the other hand, recombinant proteins can be prepared by culturing cells transformed with DNA encoding the "BG3" protein, as described below, then allowing the proteins to be expressed and collecting the expressed proteins.

It is possible for one skilled in the art to prepare an altered protein having a function equivalent to that of the naturally occurring protein (function to mediate intracellular signaling through the activation of the guanosine triphosphate binding protein) by introducing modifications, such as substitution of amino acids, in the naturally occurring human "BG3" protein (the amino acid sequence of "BG3" is shown in SEQ ID NO: 6) according to known methods . Mutations of amino acids in a protein may also occur naturally. Thus, the G protein-coupled receptor proteins of the present invention includes such mutant proteins in which the amino acid sequence is altered from that of the naturally occurring protein by substitution, deletion, addition, insertion, and so on, provided they retain a function equivalent to that of the naturally occurring protein.

Preferably, amino acids are substituted with amino acids of similar nature. For example, Ala, Val, Leu, Ile, Pro, Met, Phe, and Trp are all classified as nonpolar amino acids, and are assumed to share similar nature. Uncharged amino acids include Gly, Ser, Thr, Cys, Tyr, Asn and Gln. Asp and Glu are classified as acidic amino acids. Lys, Arg and His are classified as basic amino acids.

Methods for altering amino acids are known to those skilled in the art and include the Kunkel method (Kunkel T.A. et al., Methods Enzymol. 154:367-382 (1987)), the double primer method (Zoller M.J. and Smith M., Methods Enzymol. 154:329-350 (1987)), cassette mutagenesis (Wells et al., Gene 34:315-323 (1985)), and the megaprimer method (Sarkar G. and Sommer S.S., Biotechniques 8:404-407 (1990)). The number of mutated amino acids in a functionally equivalent protein is generally 10% or less of all the amino acids, preferably 10 amino acids or less, and more preferably 3 amino acids or less (for instance, a single amino acid).

It is also well known within the art that proteins functionally equivalent to the "BG3" protein can be obtained from DNAs homologous to the human "BG3" gene from various organisms, such homologous DNAs being isolated by way of, for example, the hybridization technique (Hanahan, D. and Meseison, M., Meth. Enzymol. 100: 333-342 (1983); Benton, W. D. and Davis, R. W., Science 196: 180-182 (1977)), and such, using the cDNA sequence for human "BG3" (the nucleotide sequence of human "BG3" is shown in SEQ ID NO: 5) or partial sequences thereof. Thus, one skilled in the art may prepare a protein that is functionally equivalent to the human "BG3" protein using DNA that hybridizes with the human "BG3" cDNA. Such proteins are included in the Gprotein-coupled receptor proteins of the present invention. Animals from which functionally equivalent proteins can be isolated include, but are not limited to, mice, rats, rabbits, cattle, dogs, monkeys, and so on. Tissues such as heart, placenta and lung from such animals are considered suitable for the isolation of cDNAs encoding a protein functionally equivalent to the "BG3" protein.

In general, DNAs encoding a protein functionally equivalent to the human "BG3" protein share high homology with the cDNA sequence of human "BG3" (SEQ ID NO: 5). The term "high homology" refers to at least more than 70%, preferably at least more than 80%, and more preferably at least more than 90% sequence homology at the nucleotide sequence level. Sequence homology may be determined by using the FASTA program.

Stringent hybridization conditions for the isolation of DNAs showing high homology with the human "BG3" cDNA are usually conducted by performing hybridization with "6X SSC, 40% formamide, 25°C" and washing with "1X SSC, 55°C". A preferable condition is "6X SSC, 40% formamide, 37°C" for hybridization and "0.2X SSC, 55°C" for washing, and more preferably, "6X SSC, 50% formamide, 37°C" for hybridization and "0.1X SSC, 62°C" for washing. Hybridization conditions with the same stringency as those described above may be achieved by one skilled in the art by suitably selecting conditions such as dilution degree of SSC, formamide concentration, temperature, and such.

The present invention also includes a partial peptide of the G protein-coupled receptor proteins described above. The partial peptides of the present invention include, for instance, a peptide corresponding to the binding site, which serves as a binding site for ligands existing in the organisms, of the protein of the present invention. Administration of such partial peptides to the living body enables competitive inhibition of the binding between the receptor protein of the present invention and its ligand, due to the antagonistic binding of the partial peptide to the native ligand. Likewise, the use of partial peptides corresponding to the binding site for the G protein makes it possible to competitively inhibit the binding of the protein of the present invention to the G protein in the cell. These partial peptides are useful as inhibitors of signal transduction mediated by the proteins of the present invention. The partial peptides of the present invention include, for example, the G protein-coupled receptor protein that has its signal sequence removed. Furthermore, the partial peptides of the present invention include, for instance, a partial peptide corresponding to the N-terminal region of the G protein-coupled receptor protein of the present invention, which can be utilized to prepare antibodies. A partial polypeptide containing the amino acid sequence specific for the protein of the present invention has a chain length of at least 7 or 8 amino acid, preferably, at least 15 amino acids, and more preferably, at least 20 amino acids.

The present invention also provides DNAs encoding the G protein-coupled receptor protein of the invention as described above, or partial peptides thereof. The DNAs encoding the G protein-coupled receptor protein of the present invention or partial peptides thereof include, but are not limited to, cDNAs, genomic DNAs, and synthetic DNAs. DNAs having any analogous nucleotide sequence, based on degeneracy of genetic code, are also included, so long as the degenerate DNA encodes a protein of the present invention.

cDNAs encoding the G protein-coupled receptor protein of the present invention can be screened, for example, by labeling the cDNA of SEQ ID NO: 5 or a fragment thereof, RNAs complementary thereto, or synthetic oligonucleotides containing a part of the cDNA sequence, with ³²P or the like, then hybridizing them to cDNA libraries derived from tissues expressing the G protein-coupled receptor protein of the present invention (e.g., tissues of heart, placenta and lung). Alternatively, cDNAs may be cloned by synthesizing oligonucleotides corresponding to the nucleotide sequence of the cDNA, and by amplifying the template cDNA from an appropriate tissue (such as tissues from heart, placenta and lung) by PCR. The genomic DNAs can be screened, for example, by labeling the cDNA of SEQ ID NO: 5 or a fragment thereof, RNAs complementary thereto, or synthetic oligonucleotides containing a part of the cDNA sequence, with ³²P or the like, and then hybridizing to a genomic DNA library. Alternatively, genomic DNAs may be cloned by synthesizing oligonucleotides corresponding to the nucleotide sequence of the cDNA, and by amplifying the genome DNA as the template by PCR. Synthetic DNAs can be prepared, for example, by chemically synthesizing oligonucleotides comprising part of the cDNA sequence of SEQ ID NO: 5, annealing them into a double strand, and then ligating them using a DNA ligase (Khorana H.G. et al., J. Biol. Chem. 251:565-570 (1976); Goeddel D.V. et al., Proc. Natl. Acad. Sci. USA 76:106-110 (1979)).

These DNAmolecules are useful for producing recombinant proteins. That is, the G protein-coupled receptor protein of the present invention may be prepared as a recombinant protein by inserting a DNA encoding the G protein-coupled receptor protein of the present invention (e.g. the DNA of SEQ ID NO: 5) into an appropriate expression vector, culturing a transformant obtained by introducing the vector into an appropriate cell, and purifying the expressed protein. Since the G protein-coupled receptor protein of the present invention is a receptor protein, it can be expressed on the cell membrane for the preparation.

Specifically, if the host is *Escherichia coli,* plasmid vectors, such as pET-3 (Rosenberg A.H. et al., Gene 56:125-135 (1987)) and pGEX-1 (Smith D.B. and Johnson K.S., Gene 67:31-40 (1988)), may be used. TheHanahanmethod (Hanahan D., J. Mol. Biol. 166:557-580 (1983)), electroporation (Dower W.J. et al., Nucleic Acids Res. 16:6127-6145 (1988)), and the like may be used for transformation of *E. coli.* If the host is a fission yeast (*Schizosaccharomyces pombe*), plasmid vectors such as pESP-1 (Lu Q. et al., Gene 200:135-144 (1997)), and so on may be used. Yeasts can be transformed, for example, by the spheroplast transformation method (Beach D. and Nurse P., Nature 290:140 (1981)), and the lithium acetate method (Okazaki K. et al., Nucleic Acids Res. 18:6485-6489 (1990)), etc.

If the host is a mammalian cell, such as Chinese Hamster ovary-derived CHO cells and human HeLa cells, vectors such as pMSG (Clontech) can be used. The recombinant DNA may be introduced into the mammalian cell by the calcium phosphate method (Graham F.L. and van derEb A.J., Virology 52:456-467 (1973)), the DEAE-dextran method (Sussman D.J. and Milman G., Mol. Cell. Biol. 4:1641-1643 (1984)), lipofection (Felgner P.L. et al., Proc. Natl. Acad. Sci. USA 84:7413-7417 (1987)), electroporation (Neumann E. et al., EMBO J. 1:841-845 (1982)), or the like.

If the host is an insect cell, baculovirus vectors such as pBacPAK8/9 (Clontech) can be used. Transformation of the insect cell can be performed according to methods known in the art, for example as described in the literature (Bio/Technology 6:47-55 (1980)).

Recombinant proteins expressed in the host cells can be purified by known methods. The proteins can also be synthesized as fusion proteins, for example, histidine residues tagged to the N-terminus, fused to glutathione-S-transferase (GST) , and such, and may be purified by binding with a metal chelating resin, or a GST affinity resin (Smith M.C. et al., J. Biol. Chem. 263:7211-7215 (1988)), respectively. For instance, when pESP-1 is used as the vector, the protein of interest is synthesized as a fusion protein with the GST; thus, the recombinant protein can be purified using a GST affinity resin. The fusion protein may be cleaved with thrombin, blood coagulation factor Xa, and such to liberate the protein of interest from the fusion protein.

In addition, the DNA encoding the G protein-coupled receptor protein of the present invention is applicable to gene therapy of diseases associated with mutations in the inventive protein. When used in gene therapy, the DNA is introduced into human cells by conventional methods, such as those utilizing retrovirus vectors (Danos O. and Mulligan R.C., Proc. Natl. Acad. Sci. USA 85:6460-6464 (1988) ; Dranoff et al. , Proc. Natl. Acad. Sci. USA90:3539-3543 (1993)), adenovirus vectors (Wickham T.J. et al., Cell 73:309-319 (1993)), etc. To administer the vector to patients, transplantation of bone marrow, subcutaneous injection, and intravenous injection can be used (Asano S., Protein Nucleic acid and Enzyme 40:2491-2495 (1995)).

The present invention also provides DNAs comprising the nucleotide sequence of SEQ ID NO: 5, or those hybridizing specifically to the complementary strand thereof, which have a chain length of at least 15 nucleotides. The DNA is preferably a DNA consisting of the nucleotide sequence of SEQ ID NO: 5, or those that hybridize specifically to the complementary strand thereof. As used herein, the term "hybridize specifically" means that no significant cross-hybridization occurs with DNAs encoding other proteins under ordinary conditions, preferably under stringent conditions for hybridization. The conditions described above can be used for the hybridization.

These DNAs of the present invention include DNAs encoding the protein of the present invention, and probes, primers, nucleotides and nucleotide derivatives (e.g. antisense oligonucleotides, ribozyme, etc.) which are capable of hybridizing specifically to a complementary DNA of the DNA.

The cDNAs as described above, that encode the G protein-coupled receptor protein of the present invention or oligonucleotides comprising a part thereof, can be used to clone the gene or cDNA, or for amplification of such by PCR. Moreover, polymorphism or abnormality of the gene and cDNA can be detected by methods such as restriction fragment length polymorphism (RFLP), and single-stranded conformational polymorphism (SSCP).

The present invention further provides antibodies that bind to the G protein-coupled receptor protein of the present invention. The antibody that binds to the G protein-coupled receptor protein of the present invention can be prepared by using methods known in the art (for instance, see Shin-Seikagaku-Jikken-Kouza (New Course on Biochemical Experiments) I: Protein I 389-406, Tokyo-Kagaku-Doujin).

For instance, polyclonal antibodies can be prepared by the procedure as follows: An appropriate dose of the proteins or peptides above is administered to an animal, such as rabbit, guinea pig, mouse, chicken and such for immunization. The proteins or peptides may be administered together with an adjuvant (such as FIA or FCA) that promotes antibody production. Administration is usually carried out every few weeks. The titer of antibodies can be increased by multiple immunizations. After the final immunization, antisera are obtained by withdrawing blood from the immunized animal. Polyclonal antibodies can be purified from antisera by, for example, performing ammonium sulfate precipitation, fractionation using anion exchange chromatography, or affinity chromatography using Protein A or immobilized antigen.

On the other hand, monoclonal antibodies, for example, are prepared as follows: The G protein-coupled receptor protein of the invention or its partial peptide is administered to animal as described above. After the final immunization, the spleen or lymph node is excised. Then, antibody-producing cells are recovered from the spleen or the lymph node, and fused with myeloma cells using, for example, polyethylene glycol and such, to produce hybridomas. Object hybridomas are screened, and then cultured. The monoclonal antibody can be prepared from the culture supernatant. The monoclonal antibody can be purified by, for example, performing ammonium sulfate precipitation, fractionation using anion exchange chromatography, or affinity chromatography using either Protein A or immobilized antigen.

Antibodies thus prepared can be used, for example, in diagnosis and antibody therapy of diseases associated with aberrant expression of the G protein-coupled receptor protein of the present invention and for detection of the expression level of the G protein-coupled receptor protein of the present invention, as well as for affinity purification of the G protein-coupled receptor protein of the invention.

It is preferable to use humanized antibodies or human antibodies for the antibody therapy. The humanized antibody, like a mouse-human chimeric antibody, can be prepared, for example, as follows: (1) isolate the gene encoding the antibody against the G protein-coupled receptor protein of the present invention from antibody-producing mouse cells; (2) replace the constant region of the H chain of the antibody with that of the human IgE; and (3) introduce into mouse myeloma J558L cells (Neuberger M.S. et al., Nature 314:268-270 (1985)). Alternatively, human antibodies can be prepared by immunizing mice whose immune systems have been replaced with that of humans with the G protein-coupled receptor protein of the present invention.

Furthermore, the present invention provides amethodof screening for ligands of the G protein-coupled receptor protein of the present invention. The method includes the steps of: (1) exposing a test compound to the G protein-coupled receptor protein of the present invention or to its partial peptide, and (2) selecting compounds that bind to the proteins or the peptides. Compounds to be tested include known compounds such as acetylcholine, adenosine, adrenaline, noradrenaline, angiotensin, bombesin, bradykinin, C5a anaphylatoxin, calcitonin, cannabinoids, chemokines, cholecystokinin, dopamine, endothelin, formylmethionylpeptide, GABA, galanin, glucagon, glutamate, glycopeptide hormone, histamine, 5-hydroxytryptophan, leucotriene, melanocortin, neuropeptide Y, neurotensin, odorant, opioid peptide, opsin, parathyroid hormone, platelet activating factor, prostanoid, somatostatin, tachykinin, thrombin, thyrotropin releasing hormone, vasopressin, oxytocin (Watson S. and Arkinstall S., The G protein Linked Receptor Facts Book, Academic Press (1994)), CGRP (calcitonin gene related protein), adrenomedullin, amylin, and serotonin; other purified proteins; gene (including library) products; extracts of tissues or cells in which the existence of the ligand is predicted (e.g. heart, lung, placenta, etc.); and culture supernatants.

The G protein-coupled receptor protein of the present invention used for the above-described screening may be as expressed intracellularly, or as expressed on the surface of the desired cells (including transformants genetically engineered to express the protein), in the form of membrane fractions of the cells, or bound to an affinity column. Test compounds used for screening may be, if necessary, suitably labeled. Labels includes, but are not limited to, radioisotope labels, fluorescence labels, and so on.

The binding of the G protein-coupled receptor protein of the present invention and a test compound can be detected through the labeling of the test compound (for instance, by measuring the radioactivity or fluorescence intensity), as well as by using, as an index, intracellular signaling mediated by the binding of the compound to the G protein-coupled receptor protein of the present invention present on the cell surface (such as G protein activation, changes in the concentration of Ca²⁺ or cAMP, phospholipase C activation, and changes in pH). The detection can be performed based on specific methods described in the literatures (Cell Calcium 14:663-671 (1993); Analytical Biochemistry 226:349-354 (1995); J. Biol. Chem. 268:5957-5964 (1993); Cell 92:573-585 (1998); Nature 393:272-273 (1998)) or in the unexamined published Japanese patent application (JP-A) No. Hei 9-268. Alternatively, the binding may be detected by measuring the activity of a reporter gene using two-hybrid system (Zervos et al., Cell 72: 223-232 (1994) ; Fritz et al., Nature 376:530-533 (1995)).

The present invention also provides a method of screening for a compound which can inhibit the binding between the G protein-coupled receptor proteins of the invention and their ligands. The method includes the steps of: (a) exposing the ligand to the G protein-coupled receptor proteins of the present invention or their partial peptides in the presence of a test compound, and detecting the binding activity between the proteins or the partial peptides and the ligand, and (b) comparing the binding activity detected in (a) with that in the absence of the test compound, and selecting a compound that reduces the binding activity. Compounds to be tested include proteins, peptides, non-peptide compounds, artificially synthesized compounds, extracts of tissues and cells, sera, and such, but are not limited thereto. The G protein-coupled receptor proteins of the present invention used for screening may be as expressed in desired cells (including transformants genetically engineered to express the proteins) or as expressed on the cell surface, in form of membrane fractions of the cells, or bound to an affinity column. If necessary, ligands for screening may be labeled appropriately. Labels include radioisotopic labels, fluorescent labels, and such, but are not limited thereto.

The binding activity between the G-protein coupled receptor proteins of the present invention, or their partial peptides, and ligands can be examined by detecting the label tagged to the ligand (for instance, by measuring the radioactivity or fluorescence intensity), as well as by using, as an index, signal transduction into the cell (such as G protein activation, changes in the concentration of Ca²⁺ or cAMP, phospholipase C activation, and changes in pH) , which are triggered by the compound binding to the G protein-coupled receptor protein of the invention on the surface of the cell. Specific examples of such methods are described in the literatures (See Cell Calcium 14:663-671 (1993); Analytical Biochemistry 226:349-354 (1995); J. Biol. Chem. 268:5957-5964 (1993); Cell 92:573-585 (1998); Nature 393:272-273 (1998)), and JP-A No. Hei 9-268.

As a result of detection, if the binding activity under the presence of a test compound is reduced compared to that in the absence of the compound (control), such a compound is determined to be an inhibitor of the binding between the G protein-coupled receptor protein of the present invention (or its partial peptides) and the ligand. Such compounds include those capable of triggering signaling into the cell through the binding to the G protein-coupled receptor protein of the invention (agonist), and those absent of such activity (antagonist). Agonists present biological activities similar to that of the ligands of the G protein-coupled receptor protein of the present invention. On the other hand, antagonists inhibit the biological activity of the ligands of the G protein-coupled receptor protein of the present invention. Accordingly, these agonists and antagonists may be useful as pharmaceutical compositions for treatment of diseases arising from disorders in the signaling pathway mediated by the G protein-coupled receptor protein of the present invention.

When a compound isolated by the screening method of the present invention is to be used as a drug, the compound may be directly administered to the patient, or it may be formulated by commonly known methods for pharmaceutical preparations. For example, the compound may be formulated by properly combining with, for example, pharmacologically acceptable carriers or media, specifically, sterilized water, physiological saline, vegetable oils, emulsifiers, suspending agents, surfactants, stabilizers, binders, lubricants, edulcorants, spices, coloring agents, and so on. Administration to patients is performed according to the methods commonly known to those skilled in the art, including intranasal, bronchial, intramuscular, and oral administrations, as well as by intra-arterial, intravenous, and subcutaneous injections, and such. Doses will vary depending on factors such as body-weight, age of the patient, and the method for administration. However, one skilled in the art can apropriately select suitable doses. If the compound can be encoded by a DNA, such a DNA may be integrated into a gene therapy vector for use in gene therapy as described above. Doses and administration methods will vary depending on factors such as body-weight, age of the patient, state of the disease, and so on. However, one skilled in the art can appropriately select suitable doses and methods.

Furthermore, the present invention provides a kit to screen for a compound that inhibits binding between the G protein-coupled receptor protein of the present invention and its ligand. One feature of the kit is that it contains the G protein-coupled receptor protein of the present invention or a partial peptide thereof. The G protein-coupled receptor protein of the present invention (or a partial peptide thereof) in the kit of the present invention may take its form as expressed in the desired cells (including transformants genetically engineered to express the protein) or as expressed on the cell surface, or it may be in a form of membrane fractions of the cell, or bound to an affinity column. In addition to a receptor protein sample as described above, the kit of the invention may include a ligand sample (both labeled and unlabeled) , buffer for the receptor protein-ligand reaction, washing solution, and so on. Labels of the ligands include, for instance, radioactive labels and fluorescent labels. The kit of the present invention may be used, for example, according to the description of JP-A No. Hei 9-268.

### Brief Description of the Drawings

Figure 1 shows the hydrophobicity plot of the human "BG3" protein. In the Figure, numbers I to VII indicate the seven hydrophobic regions (transmembrane regions) characteristic of G protein-coupled receptor proteins. The numbers on the top indicate the positions of the amino acid residues in the "BG3" protein.
Figure 2 is an electrophoresis photograph of the results of Northern blot analysis of the tissue specific expression of the human "BG3" gene.
Figure 3 is an electrophoresis photograph of the results of Southern blot analysis of the human "BG3" gene.

### Best Mode for Carrying out the Invention

The present invention is illustrated in detail below with reference to the examples, but is not to be construed as being limited thereto.

### Example 1: Isolation of the human G protein-coupled receptor gene

### <1> Manipulation of the Gene Trapper and design of the probe.

The G protein-coupled receptors are structurally characterized by seven transmembrane regions, and the DNA and amino acid sequences of the transmembrane regions and their adjacent regions are well conserved. Thus, sense primer Fg (5'-BATNGCCAAC CTBKCCTTCT C-3' / SEQ ID NO: 1; 21mer, Degeneracy 72) (B= G, T or C, K= G or T, and N= A, C, G or T) was synthesized on the basis of the comparison of the DNA sequence of the second transmembrane domain conserved among the known G protein-coupled receptors, namely the mouse neuropeptide Y receptor Y1 (GenBank Accession Number Z18280) , ratY1 (Z11504) , humanY1 (M84755), mouse neuropeptide Y receptor Y4 (U40189), rat Y4 (Z681 80), human Y4 (Z66526), and mouse neuropeptide Y receptor Y6 (U58367). Mixed nucleotides were used to increase the sequence identity with different receptor cDNAs,

Gene Trapper (GIBCO BRL) was used in the Biotin-Avidin Capture method. In this method, the library of plasmids containing the fl ori sequence was digested to a single-stranded plasmid library, using Gene II and Exo III nuclease. A sense primer for the gene of interest was synthesized and biotinylated to provide a probe. The biotinylated probe was allowed to hybridize with the single-stranded plasmid library, and single-stranded plasmid DNA to which the probe was specifically bound was collected using streptavidin-coated magnetic beads and then washed. Double-stranded plasmid DNA was synthesized again from this single-stranded plasmid DNA by using the same primer. XL1 Blue MRF' cells (host *E*. *coli* cells) were transformed with the DNA thus synthesized using *E. coli* pulser (BIORAD). The procedure was performed according to the details of the protocol provided by the manufacturer.

384 clones were randomly selected from the clones obtained by the Gene Trapper method using a human fetal brain cDNA library (GIBCO BRL) as the screening source, inoculated in the LB medium containing 100 µg/ml of ampicillin and cultured overnight at 37°C. The plasmid DNAs were purified with BioRobot9600 (QIAGEN) using the QIA Prep 96 Turbo BioRobot Kit (QIAGEN). For sequencing, the sequencing reaction was carried out using ABI PRISM877 (ABI) with BigDye Primer Cycle Sequencing Ready Reaction Kit (ABI), followed by electrophoresis using automated fluorescence-based DNA sequencer ABI PRISM 377 DNA Sequencer (ABI). Partial nucleotide sequence at the 5' terminal region of each clone was determined, which was further analyzed by homology searches (BLAST search) in various databases. As a result, inventors discovered the clone GFgHG0360 (SEQ ID NO: 2), which appeared to encode a novel G protein-coupled receptor that showed significant homology to known G protein-coupled receptors. The novel G protein-coupled receptor predicted to be encoded by the clone was designated "BG3".

### <2> Sequencing

The nucleotide sequence of clone GFgHG0360 was determined using the shotgun cloning method (Sambrook et al., Molecular Cloning: A laboratory manual 2^{nd} edition (1989)). Biorupter (Tosyo Denki Co.), a closed ultrasonic homogenizer for biomaterials, was used for fragmentation of the cDNA, and the DNA fragments were fractionated by electrophoresis on a 1.2% agarose gel. The gel slice containing the DNA fragments about 0.5-1.0 kb in length was cut out, and the DNA fragments were purified with Gene Clean (Bio101), blunt-ended with T4 DNA polymerase (TaKaRa), and subcloned. Clones thus obtained were subjected to the sequencing reaction. The DNA sequences obtained were analyzed using the sequencing softwares Sequencher (Hitachi Software Engineering) and RASERGENE (DNASTAR). The results showed that the sequence contained a poly (A) tail at the 3' terminus but lacked a stop codon upstream of the initiation codon at the 5'-side, suggesting that the sequence does not contain the complete open reading frame.

### Example 2: Northern blot analysis of human BG3

To study the expression pattern of the receptor mRNA encoding the "BG3" protein expressed in human tissues, Northern blot analysis was performed using human MTN Blot I (CLONTECH).

Based on the previously determined nucleotide sequence of clone GFgHG0360, sense and antisense primers having the following sequences were synthesized: BG3F01: 5'-CCACGCCAACCTGTCCTTCGC-3'/ SEQ ID NO: 3 and BG3R04: 5′-CTGCTCGTGAGCGACCAGACC-3′/ SEQ ID NO: 4. Conditions for the PCR were: 30 cycles of 94°C for 240 seconds, 94°C for 10 seconds, 55°C for 5 seconds, and 74°C for 60 seconds. PCR was performed using a reaction mixture containing 1X Buffer (standard buffer supplied with the kit), 0.2 mM dNTPs, 1 mM of each primer, 5 ng of template DNA (GFgHG0360), and 0.25 Unit/ml KOD' polymerase (TOYOBO). The DNA band, having a size of about 0.7 kb, was cut out from the agarose gel after electrophoresis and purified with Gene Clean (Bio 101) to provide the template for preparing the probes.

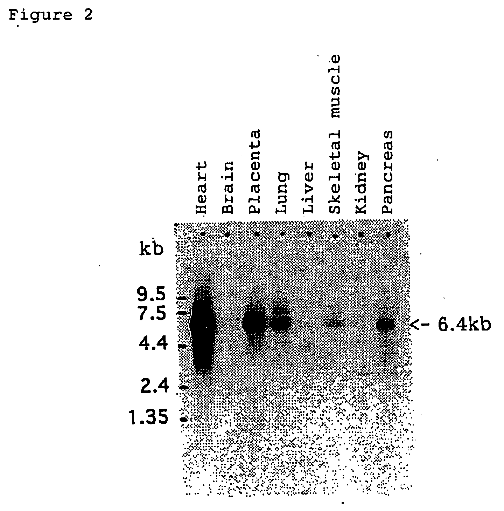

The probe was prepared by labeling with [α-³²P]dCTP (Amersham) according to the protocol supplied with the Prime-It II Random Primer Labeling Kit (Stratagene). Conditions for hybridization and washing were determined according to the manufacturers' protocol provided with the Human MTN Blot I (CLONTECH). Final washing was done with 0.1X SSC-0.1% SDS for 30 minutes at 55°C. Northern blot analysis revealed signals in all the tissues tested (heart, brain, placenta, lung, liver, skeletal muscle, kidney and pancreas). Among them, particularly intense signal was detected in heart, placenta and lung (Fig. 2).

### Example 3: Isolation of the full-length clone of human "BG3"

To efficiently screen for the full-length cDNA clone of human "BG3", the ZAP II cDNA library (Stratagene) derived from human heart, whose abundant expression of human "BG3" mRNA was confirmed by the Northern blot analysis, was used for the screening. The same human "BG3" as the Northern blot analysis, was used for the screening, and 24 positive clones were obtained. A single cDNA clone having an insert of 5.4 kb that was predicted to encode the 5'-upstream region, the complete open reading frame and the 3'-downstream region of "BG3" was obtained as the result of the 5'- and 3'-sequencing of the clones.

This clone *("E. coli* hBG3-17") was deposited in the National Institute of Bioscience and Human-Technology, Agency of Industrial Science and Technology.
Name of the depositary institution:
The National Institute of Bioscience and Human-Technology, Agency of Industrial Science and Technology, Ministry of International Trade and Industry.
Address of the depositary institution:
1-1-3 Higashi, Tsukuba, Ibaraki 305-8566, Japan.
Date of deposit (original deposit): March 15, 1999.
Accession Number: FERM BP-7070

The partial nucleotide sequences of the 5' region of GFgHG0360 and the 24 clones, which were obtained by the screening of the human heart ZAP II cDNA library, were aligned to synthesize the primers for sequencing. BigDye Terminator Cycle Sequencing Ready Reaction Kit (ABI) was used to determine the nucleotide sequence of the human "BG3" clone (*E*. *colihBG3-17*). The DNA sequence determined was analyzed using DNA sequencing softwares Sequencher (Hitachi Software Engineering) and RASERGENE (DNASTAR).

The result revealed that the cDNA (SEQ ID NO: 5) contained a translation frame (open reading frame) encoding a protein consisting of 874 amino acid residues (SEQ ID NO: 6). This cDNA appeared to contain the complete ORF, since it comprised a poly(A) tail at the 3' end and a termination codon upstream of-the initiation codon at the 5' terminal region.

According to the analysis of the amino acid sequence of this DNA, seven transmembrane regions, from the first to the seventh, in the hydrophobic domain characteristic for the G protein-coupled receptor proteins were identified on the hydrophobicity plots (Fig. 1). Further, a signal peptide rich in hydrophobic residues was also identified at the N-terminus.

### Example 4: Southern blot analysis of "BG3"

Analysis was performed to determine whether genes encoding the proteins functionally equivalent to the "BG3" protein were conserved among species. ZOO-BLOT (CLONTECH), on which genomic DNA from human, monkey, rat, mouse, dog, cattle, rabbit, chicken and yeast completely digested with endonuclease EcoRI were blotted, was used for the Southern blot analysis.

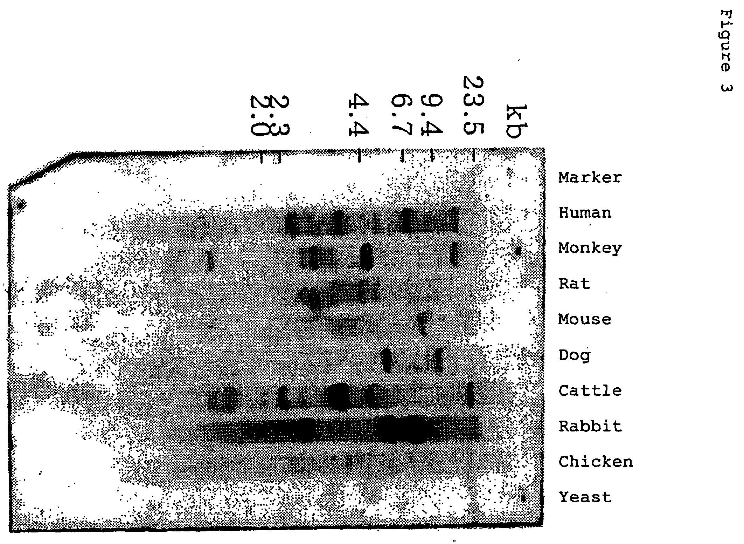

A probe labeled with ³²P was prepared as described above in Example 2, using the same primers that were used in the Northern blot analysis of "BG3". Hybridization and washing were carried out according to the protocol of ZOO-BLOT (CLONTECH). Final washing was performed in 0.1X SSC-0.1% SDS for 30 minutes at 55°C. Multiple band signals were detected in all the mammals tested including human, monkey, rat, mouse, dog, cattle, and rabbit by the Southern blot analysis, which was performed against genomic DNAs of human, monkey, rat, mouse, dog, cattle, rabbit, chicken, and yeast (Fig. 3).

The fact that signals were detected in the genomic DNA from mammals by the hybridization using the DNA encoding the human "BG3" protein as a probe indicated that a gene encoding the protein functionally equivalent to the "BG3" protein was conserved among species. This further suggested that the "BG3" protein played an important role and was a potential target protein in developing therapeutic agents for various diseases.

The result, that multiple bands were detected by the Southern blot analysis, suggested that the DNA encoding the protein with equivalent function as the "BG3" protein have a structure consisting of multiple exons and introns on the chromosome. Such structure may allow synthesis of different splicing forms of the "BG3" protein from a single gene encoding the "BG3" protein by way of various alternative splicing. Alternatively, it may suggest the existence of genes encoding other proteins that have functions equivalent to the "BG3" protein, or genes encoding different proteins having partial homology to the "BG3" protein but whose function is different from that of the "BG3" protein.

### Industrial Applicability

The present invention providesa novel G protein-coupled receptor, protein which is expressed in various tissues, as well as the DNA encoding the protein. Use of the inventive receptor protein makes it possible to screen for ligands and candidate medicinal compounds. It is expected that these ligands and candidate compounds may be used, for example, in the diagnosis, treatment, and such of diseases associated with disorders in the signal transduction system mediated by the G protein-coupled receptor protein of the invention.

## Claims

1. A DNA encoding a guanosine triphosphate binding protein-coupled receptor protein, wherein said DNA is selected from one of the following:
(a) a DNA encoding a protein consisting of the amino acid sequence of SEQ ID NO: 6;
(b) a DNA comprising a coding region of the nucleotide sequence of SEQ ID NO: 5;
(c) a DNA encoding a protein consisting of the amino acid sequence of SEQ ID NO: 6, in which one or more amino acids are substituted, deleted, inserted and/or added, ; and
(d) a DNA that hybridizes under stringent conditions with a DNA consisting of the nucleotide sequence of SEQ ID NO: 5.

2. A DNA encoding a partial peptide of a guanosine triphosphate binding protein-coupled receptor protein consisting of the amino acid sequence of SEQ ID NO: 6.

3. A vector containing the DNA of claims 1 or 2.

4. A transformant having the DNA of claims 1 or 2, or the vector of claim 3.

5. A protein or peptide encoded by the DNA of claims 1 or 2.

6. A method for producing the protein or peptide of claim 5, which comprises the steps of: culturing the transformant of claim 4 and collecting the expressed protein from the transformant or culture supernatant thereof.

7. A method of screening for a ligand that binds to the protein or peptide of claim 5, wherein the method comprises the steps of:
(a) contacting a test compound with the protein or peptide of claim 5; and
(b) selecting the compound that binds to said protein or peptide.

8. A method of screening for a compound that has an inhibitory activity on the binding between the protein or peptide of claim 5 and a ligand thereof, wherein the method comprises the steps of:
(a) detecting the binding activity of the protein or peptide of claim 5 to its ligand by contacting said protein or peptide with the ligand in the presence of a test compound; and
(b) selecting the compound that reduces the binding activity of said protein or peptide to the ligand, by comparing the binding activity detected in step (a) with that detected in the absence of the test compound.

9. A kit for screening for a compound that inhibits the binding of the protein or peptide of claim 5 to its ligand, wherein said kit comprises the protein or peptide of claim 5.

10. An antibody that binds to the protein of claim 5;

11. A DNA that hybridizes to the DNA consisting of the nucleotide sequence of SEQ ID NO: 5 or to a complementary strand thereof, wherein
said DNA has a length of at least 15 nucleotides.
